(19) Europäisches Patentamt
European Patent Office
Office européen des brevets

(11) Veröffentlichungsnummer: **0 559 000 A2**

# (12) EUROPÄISCHE PATENTANMELDUNG

(21) Anmeldenummer: **93102465.7**

(22) Anmeldetag: **17.02.93**

(51) Int. Cl.[5]: **C07D 249/08**, C07D 303/46, A01N 43/653

(30) Priorität: **02.03.92 DE 4206529**

(43) Veröffentlichungstag der Anmeldung: **08.09.93 Patentblatt 93/36**

(84) Benannte Vertragsstaaten: **BE CH DE DK ES FR GB IT LI NL**

(71) Anmelder: **BAYER AG**

**D-51368 Leverkusen(DE)**

(72) Erfinder: **Scherkenbeck, Jürgen, Dr.**
**Auf dem Bruch 49**
**W-5090 Leverkusen 3(DE)**
Erfinder: **Himmler, Thomas, Dr.**
**Schöne Aussicht 1b**
**W-5068 Odenthal(DE)**
Erfinder: **Hagemann, Hermann, dr.**
**Kandinskystrasse 52**
**W-5090 Leverkusen 1(DE)**
Erfinder: **Dutzmann, Stefan, Dr.**
**Kosenberg 10**
**W-4010 Hilden 1(DE)**
Erfinder: **Dehne, Heinz-Wilhelm, Dr.**
**Krischer Strasse 81**
**W-4019 Monheim(DE)**
Erfinder: **Hänssler, Gerd, Dr.**
**Am Arenzberg 58a**
**W-5090 Leverkusen 1(DE)**

(54) **Azolylmethyl-cyclopropyl-Derivate.**

(57) Neue Azolylmethyl-cyclopropyl-Derivate der Formel

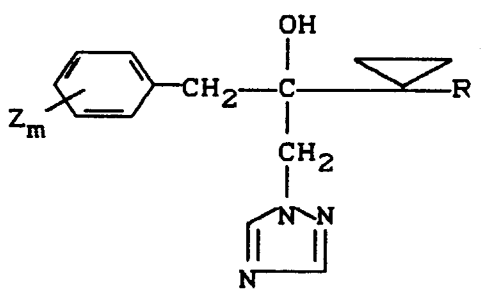

(I)

in welcher

R für Cyano oder eine Gruppe der Formel

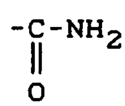

steht,

Z für Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen

EP 0 559 000 A2

und 1 bis 5 Halogenatomen oder für Alkoximinoalkyl mit 1 bis 4 Kohlenstoffatomen in der Alkoxygruppe und 1 bis 4 Kohlenstoffatomen in der Alkylgruppe steht und

m für die Zahlen 0, 1 oder 2 steht,

sowie deren Säureadditions-Salze und Metallsalz-Komplexe,
ein Verfahren zur Herstellung der neuen Stoffe und deren Verwendung als Fungizide.

Neue Oxirane der Formel

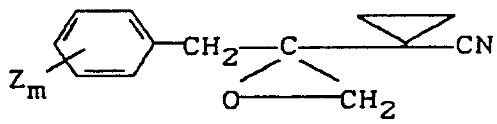

(II)

in welcher

Z und m die oben angegebenen Bedeutungen haben,

ein Verfahren zu deren Herstellung und deren Verwendung als Zwischenprodukte zur Synthese von Azolylmethyl-cyclopropyl-Derivaten der Formel (I).

Die vorliegende Erfindung betrifft neue Azolylmethyl-cyclopropyl-Derivate, ein Verfahren zu deren Herstellung und deren Verwendung als Fungizide.

Es ist bereits bekannt geworden, daß bestimmte Hydroxyethyl-azolyl-Derivate fungizide Eigenschaften besitzen (vergl. EP-OS 0 106 515). So lassen sich zum Beispiel 2-(4-Chlorphenyl)-1-(1,2,4-triazol-1-yl)-3-cyano-3-methyl-butan-2-ol und 2-(2,4-Dichlorphenyl)-1-(1,2,4-triazol-1-yl)-3-methyl-3-carboxamido-butan-2-ol zur Bekämpfung von Pilzen verwenden, Die Wirkung dieser Stoffe ist gut, läßt aber bei niedrigen Aufwandmengen in manchen Fällen zu wünschen übrig.

Es wurden nun neue Azolylmethyl-cyclopropyl-Derivate der Formel

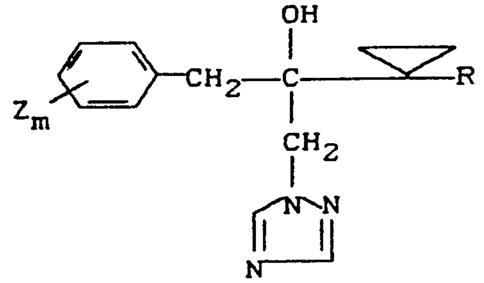

(I)

in welcher

R für Cyano oder eine Gruppe der Formel

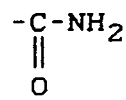

steht,

Z für Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen oder für Alkoximinoalkyl mit 1 bis 4 Kohlenstoffatomen in der Alkoxygruppe und 1 bis 4 Kohlenstoffatomen in der Alkylgruppe steht und

m für die Zahlen 0, 1 oder 2 steht,

sowie deren Säureadditions-Salze und Metallsalz-Komplexe gefunden.

Weiterhin wurde gefunden, daß man Azolylmethyl-cyclopropyl-Derivate der Formel (I) sowie deren Säureadditions-Salze und Metallsalz-Komplexe erhält, wenn man in einer ersten Stufe Oxirane der Formel

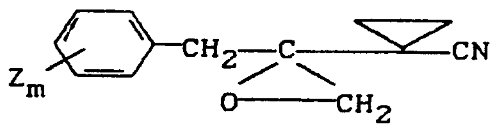

(II)

in welcher

Z und m die oben angegebenen Bedeutungen haben,

mit 1,2,4-Triazol der Formel

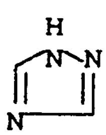

(III)

gegebenenfalls in Gegenwart eines Säurebindemittels und in Gegenwart eines Verdünnungsmittels umsetzt, und gegebenenfalls in einer zweiten Stufe die dabei entstehenden Verbindungen der Formel

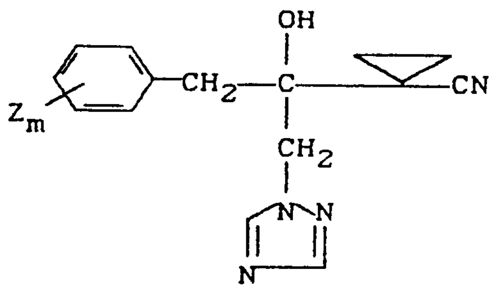

(Ia)

in welcher

Z und m die oben angegebenen Bedeutungen haben,

mit Wasserstoffperoxid in Gegenwart eines inerten organischen Verdünnungsmittels und in Gegenwart einer wäßrigen Alkalilauge sowie in Gegenwart eines Phasentransfer-Katalysators behandelt, und gegebenenfalls anschließend an die so erhaltenen Verbindungen der Formel (I) eine Säure oder ein Metallsalz addiert.

Schließlich wurde gefunden, daß die neuen Azolylmethyl-cyclopropyl-Derivate der Formel (I) sowie deren Säureadditions-Salze und Metallsalz-Komplexe sehr gute fungizide Eigenschaften aufweisen.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Überraschenderweise besitzen die erfindungsgemäßen Stoffe bessere fungizide Eigenschaften als das 2-(4-Chlor-phenyl)-1-(1,2,4-triazol-1-yl)-3-cyano-3-methyl-butan-2-ol und das 2-(2,4-Dichlorphenyl)-1-(1,2,4-triazol-1-yl)-3-methyl-3-carboxamido-butan-2-ol, welches konstitutionell ähnliche, vorbekannte Wirkstoffe gleicher Wirkungsrichtung sind.

Die erfindungsgemäßen Azolylmethylcyclopropyl-Derivate sind durch die Formel (I) allgemein definiert.

R steht für Cyano oder eine Gruppe der Formel

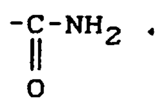

Z steht vorzugsweise für Fluor, Chlor, Alkyl mit 1 oder 2 Kohlenstoffatomen, Halogenalkyl mit 1 oder 2 Kohlenstoffatomen und 1 bis 3 gleichen oder verschiedenen Halogenatomen, Alkoxy mit 1 oder 2 Kohlenstoffatomen, Halogenalkoxy mit 1 oder 2 Kohlenstoffatomen und 1 bis 3 gleichen oder verschiedenen Halogenatomen oder für Alkoximinoalkyl mit 1 oder 2 Kohlenstoffatomen in der Alkoxygruppe und 1 oder 2 Kohlenstoffatomen in der Alkylgruppe.

m steht vorzugsweise für die Zahlen 1 oder 2.

Z steht besonders bevorzugt für Fluor, Chlor, Methyl, Trifluormethyl, Methoxy, Trifluormethoxy, Methoximino-methyl oder Ethoximinomethyl.

Bevorzugte erfindungsgemäße Stoffe sind auch Additionsprodukte aus Säuren und denjenigen Azolylmethyl-cyclopropyl-Derivaten der Formel (I), in denen R, Z und m die als bevorzugt angegebenen Bedeutungen haben.

Zu den sauren, die addiert werden können, gehören vorzugsweise Halogenwasserstoffsäuren, wie z.B. die Chlorwasserstoffsäure und die Bromwasserstoffsäure, insbesondere die Chlorwasserstoffsaure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Azolylmethyl-cyclopropyl-Derivaten der Formel (I), in denen R, Z und m die als bevorzugt angegebenen Bedeutungen haben.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten,

die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Azolylmethyl-cyclopropyl-Derivate der Formel (I) seien die in der folgenden Tabelle aufgeführten Stoffe genannt.

Tabelle 1

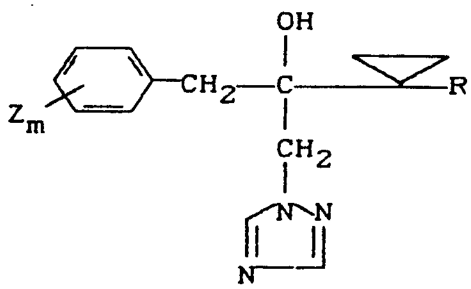

(I)

| $Z_m$ | R | $Z_m$ | R |
|---|---|---|---|
| 4-Cl | -CN | 4-$CF_3$ | -CO-$NH_2$ |
| 4-Cl | -CO-$NH_2$ | 2-$OCH_3$ | -CN |
| 4-F | -CO-$NH_2$ | 2-$OCH_3$ | -CO-$NH_2$ |
| 4-F | -CN | 2-$OCF_3$ | -CN |
| 2,4-$Cl_2$ | -CN | 2-$OCF_3$ | -CO-$NH_2$ |
| 2,6-$Cl_2$ | -CN | 2-$OCHF_2$ | -CN |
| 2-$CH_3$ | -CN | 2,4-$F_2$ | -CN |
| 2-$CH_3$ | -CO-$NH_2$ | 2,4-$F_2$ | -CO-$NH_2$ |
| 2-$CF_3$ | -CN | 2-CH=N-$OCH_3$ | -CN |
| 2-$CF_3$ | -CO-$NH_2$ | 4-CH=N-$OCH_3$ | -CN |
| 4-$CF_3$ | -CN | 4-CH=N-$OCH_3$ | -CO-$NH_2$ |

Verwendet man 2-(2-Fluorbenzyl)-2-(1-cyano-cyclopropyl)-oxiran und 1,2,4-Triazol als Ausgangsstoffe, so kann der Verlauf des erfindungsgemäßen Verfahrens durch das folgende Formelschema veranschaulicht werden:

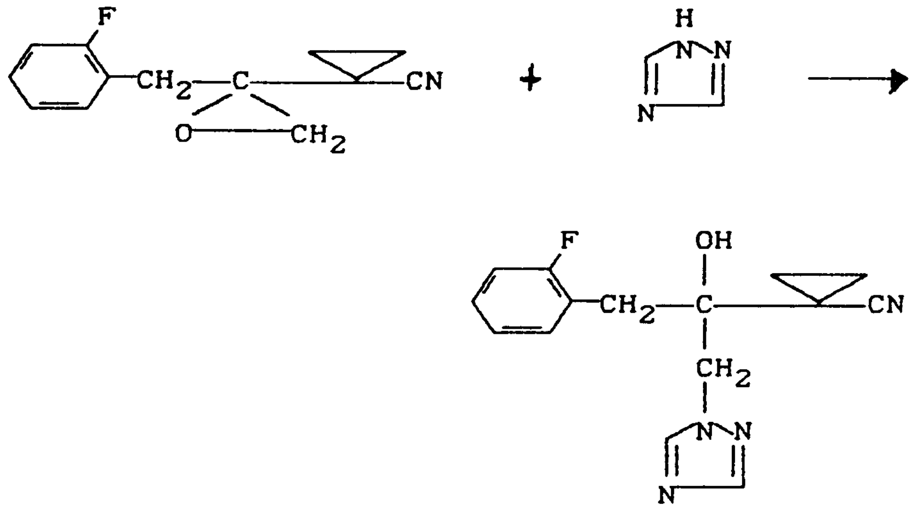

Verwendet man 1-(2-Fluorphenyl)-2-(1-cyano-cyclopropyl)-3-(1,2,4-triazol-1-yl)-propan-2-ol als Ausgangsstoff und behandelt dieses mit wäßriger Wasserstoffperoxid-Lösung in Gegenwart von Tetrabutylammonium-hydrogensulfat sowie wäßriger Natronlauge und Dichlormethan, so kann der Verlauf der zweiten Stufe des erfindungsgemäßen Verfahrens durch das folgende Formelschema veranschaulicht werden:

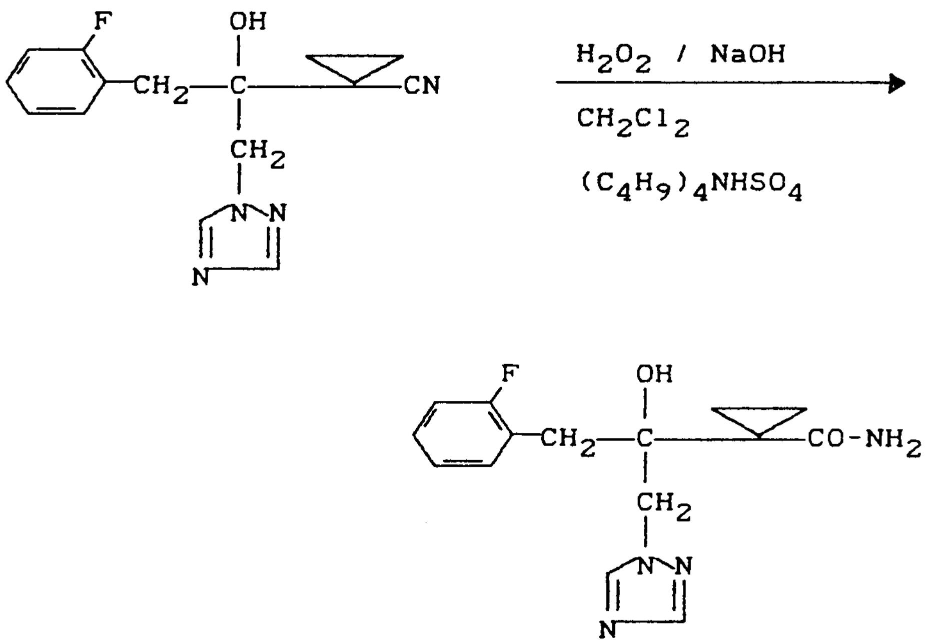


Die bei dem erfindungsgemäßen Verfahren als Ausgangsstoffe benötigten Oxirane der Formel (II) sind noch nicht bekannt. Sie lassen sich herstellen, indem man Benzylketone der Formel

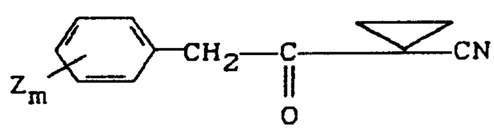
(IV)

in welcher
Z und m die oben angegebenen Bedeutungen haben,
entweder
α) mit Dimethyloxosulfonium-methylid der Formel

$$\overset{\delta^{\oplus}}{S}\overset{\delta^{\ominus}}{}$$

$$(CH_3)_2SOCH_2 \qquad (V)$$

oder
β) mit Dimethylsulfonium-methylid der Formel

$$\delta^{\oplus}\delta^{\ominus}$$

$$(CH_3)_2S\ CH_2 \qquad (VI)$$

in Gegenwart eines Verdünnungsmittels umsetzt.
Die Benzylketone der Formel (IV) lassen sich herstellen, indem man
a) in einer ersten Stufe Benzylchloride der Formel

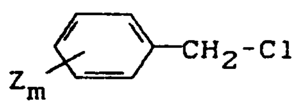
(VII)

in welcher
Z und m die oben angegebenen Bedeutungen haben,
mit einem Überschuß an Zinkpulver in Gegenwart eines Verdünnungsmittels, wie zum Beispiel Ethylenglykol-dimethylether oder Tetrahydrofuran, bei Temperaturen zwischen 50 °C und 150 °C unter Schutzgasatmosphäre umsetzt, das überschüssige Zinkpulver abtrennt und dann
b) in einer zweiten Stufe die entstandenen Benzylderivate der Formel

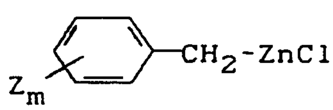
(VIII)

in welcher
Z und m die oben angegebenen Bedeutungen haben,
mit 1-Cyano-cyclopropyl-carbonsäure-chlorid der Formel

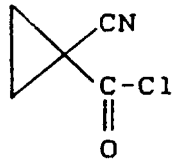
(IX)

in Gegenwart eines Palladiumkatalysators, wie zum Beispiel Bis-(triphenylphosphin)-palladium-(II)-chlorid, und in Gegenwart eines Verdünnungsmittels, wie zum Beispiel Ethylenglykol-dimethylether oder Tetrahydrofuran, bei Temperaturen zwischen 20 °C und 100 °C unter Schutzgasatmosphäre umsetzt.

Die bei der Durchführung des obigen Verfahrens als Ausgangsstoffe benötigten Benzylchloride der Formel (VII) und das als Reaktionskomponente benötigte 1-Cyano-cyclopropyl-carbonsäure-chlorid der Formel (IX) sind bekannt oder lassen sich nach prinzipiell bekannten Verfahren herstellen.

Das bei der Durchführung der Variante (α) des Verfahrens zur Herstellung von Oxiranen der Formel (II) als Reaktionskomponente benötigte Dimethyloxosulfonium-methylid der Formel (V) ist bekannt (vergl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, oder durch Umsetzung von Trimethyloxosulfoniumchlorid mit wäßriger Natronlauge, jeweils in Gegenwart eines Verdünnungsmittels erzeugt.

Das bei der Durchführung der Variante (β) des Verfahrens zur Herstellung von Oxiranen der Formel (II) außerdem als Reaktionskomponente in Betracht kommende Dimethylsulfonium-methylid der Formel (VI) ist ebenfalls bekannt (vgl. Heterocycles 8, 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des obigen Verfahrens zur Herstellung von Oxiranen der Formel (II) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des obigen Verfahrens zur Herstellung von Oxiranen der Formel (II) in einem größeren Bereich variiert werden. Im allgemeinen arbeitet man bei Temperaturen zwischen 0 °C und 100 °C, vorzugsweise zwischen 10 °C und 60 °C.

Bei der Durchführung des obigen Verfahrens zur Herstellung von Oxiranen der Formel (II) setzt man auf 1 Mol an Benzylketon der Formel (IV) im allgemeinen 1 bis 3 Mol an Dimethyloxosulfonium-methylid der Formel (V) bzw. an Dimethylsulfonium-methylid der Formel (VI) ein. Die Isolierung der Oxirane der Formel (II) erfolgt nach üblichen Methoden.

Als Säurebindemittel kommen bei der Durchführung der ersten Stufe des erfindungsgemäßen Verfahrens alle üblichen anorganischen und organischen Basen in Frage. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium-und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung der ersten Stufe des erfindungsgemäßen Verfahrens alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 0 °C und 200 °C, vorzugsweise zwischen 50 °C und 150 °C.

Bei der Durchführung der ersten Stufe des erfindungsgemäßen Verfahrens setzt man vorzugsweise auf 1 Mol an Oxiran der Formel (II) 1 bis 4 Mol an Azol der Formel (III) und 1 bis 2 Mol an Base ein. Die Isolierung der Endprodukte erfolgt in üblicher Weise.

Bei der Durchführung der zweiten Stufe des erfindungsgemäßen Verfahrens wird Wasserstoffperoxid in Form einer wäßrigen Lösung eingesetzt. Im allgemeinen verwendet man eine 30 %ige wäßrige Lösung von Wasserstoffperoxid.

Als Phasentransfer-Katalysatoren kommen bei der Durchführung der zweiten Stufe des erfindungsgemäßen Verfahrens alle für derartige Zwecke üblichen Reaktionsbeschleuniger in Betracht. Vorzugsweise verwendbar sind Tetrabutylammoniumchlorid, Tetrabutylammonium-hydrogen-sulfat, Benzyl-triethyl-ammonium-chlorid und Benzyl-triethyl-ammonium-bromid.

Als wäßrige Alkalilaugen kommen bei der Durchführung der zweiten Stufe des erfindungsgemäßen Verfahrens vorzugsweise Natronlauge und Kalilauge in Betracht.

Als organische Verdünnungsmittel kommen bei der Durchführung der zweiten Stufe des erfindungsgemäßen Verfahrens vorzugsweise aromatische Kohlenwasserstoffe, wie Benzol oder Toluol, oder halogenierte aliphatische Kohlenwasserstoffe, wie Dichlormethan oder Chloroform, in Frage.

Die Reaktionstemperaturen können auch bei der Durchführung der zweiten Stufe des erfindungsgemäßen Verfahrens innerhalb eines größeren Bereiches variiert werden, Im allgemeinen arbeitet man bei Temperaturen zwischen -20°C und +60°C, vorzugsweise zwischen 0°C und 50°C.

Bei der Durchführung der zweiten Stufe des erfindungsgemäßen Verfahrens geht man so vor, daß man auf 1 Mol an Verbindung der Formel (Ia) einen Überschuß an wäßriger Wasserstoffperoxid-Lösung und eine katalytische Menge eines Phasen-transfer-Katalysators sowie wäßrige Alkalilauge in einem geringen Überschuß einsetzt. Die Aufarbeitung erfolgt nach üblichen Methoden.

Die erfindungsgemäßen Azolylmethyl-cyclopropyl-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;

Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Leptosphaeria nodorum, Cochliobolus sativus, Pyrenophora teres, Pseudocercosporella herpotrichoides, Erysiphe und Fusarium-Arten. Außerdem zeigen die erfindungsgemäßen Stoffe eine sehr gute Wirkung gegen Venturia, Sphaerotheca und Botrytis.

Die erfindungsgemäßen Stoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je kg Saatgut, vorzugsweise 0,01 bis 10 g benötigt. Bei der Behandlung des Bodens sind

Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Stoffe geht aus den folgenden Beispielen hervor.

Herstellungsbeispiele

Beispiel 1

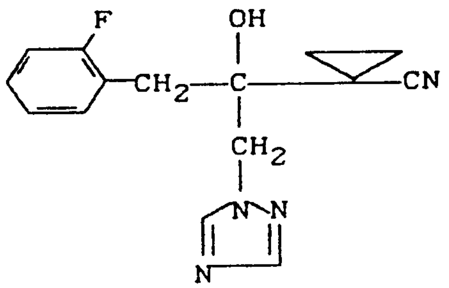

(I-1)

In eine Lösung von 0,23 g (2,1 mmol) Kalium-tert.-butylat und 2,15 g (31,2 mmol) 1,2,4-Triazol in 10 ml Dimethylformamid werden 2,26 g (10,4 mmol) 2-(2-Fluorbenzyl)-2-(1-cyanocyclopropyl)-oxiran in 10 ml Dimethylformamid bei 80 °C unter Rühren eingetropft. Nach beendeter Zugabe wird das Reaktionsgemisch 4 Stunden bei 80 °C nachgerührt. Anschließend wird unter vermindertem Druck eingeengt, und der Rückstand wird in Ethylacetat-Wasser aufgenommen. Die wäßrige Phase wird dreimal mit Ethylacetat extrahiert. Die vereinigten organischen Extrakte werden über Natriumsulfat getrocknet, unter vermindertem Druck eingeengt und an Kieselgel mit dem Laufmittel Cyclohexan-Ethylacetat = 1 : 1 chromatographiert. Dabei werden 1,37 g (46 % der Theorie) an 1-(2-Fluorphenyl)-2-(1-cyanocyclopropyl)-3-(1,2,4-triazol-1-yl)-propan-2-ol isoliert.
$^1$H-NMR (200 MHz, $CDCl_3$) : $\delta$ = 0,4-1,1(m;4H), 3,2 (AB-System; 2H), 4,12 (s; 1H), 4,5 (AB-System; 2H), 7,05-7,45 (m; 4H), 7,0 (s; 1H), 8,33 (s; 1H).

Herstellung von Ausgangssubstanzen:

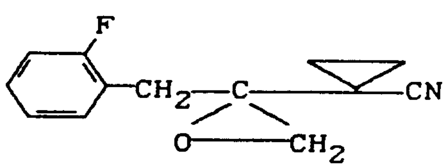

(II-1)

In eine Suspension von 6,4 g (31,5 mmol) 1-Cyanocyclopropyl-2-fluorbenzyl-keton und 4,46 g (34,6 mmol) Trimethylsulfoxoniumchlorid in 30 ml Toluol werden 13,8 ml 45 %ige wäßrige Natronlauge bei Raumtemperatur innerhalb 15 Minuten eingetropft. Nach beendeter Zugabe wird das Reaktionsgemisch drei Stunden bei Raumtemperatur und eine Stunde bei 40 °C nachgerührt. Das Reaktionsgemisch wird mit Wasser verdünnt, und die Phasen werden getrennt. Die wäßrige Phase wird dreimal mit Cyclohexan extrahiert. Die vereinigten organischen Extrakte werden einmal mit Wasser gewaschen, über Natriumsulfat getrocknet und unter vermindertem Druck eingeengt. Das Rohprodukt wird ohne zusätzliche Reinigung für die weitere Umsetzung verwendet.

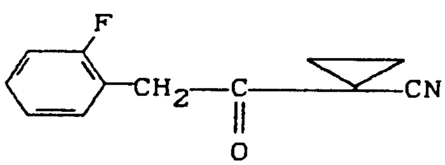

(IV-1)

Ein Gemisch aus 32,7 g (0,5 Mol) Zinkpulver, 50,6 g (0,35 Mol) 2-Fluor-benzyl-chlorid und 375 ml trockenem Ethylenglykol-dimethylether wird unter Stickstoffatmosphäre 2 Stunden unter Rückfluß erhitzt. Anschließend wird das Reaktionsgemisch unter Stickstoff filtriert. Das Filtrat wird mit 38,9 g (0,3 Mol) 1-Cyano-cyclopropyl-carbonsäure-chlorid und 21 mg (0,01 Mol-%) Bis-(triphenylphosphin)-palladium(II)-chlorid versetzt und unter Stickstoffatmosphäre 2 Stunden unter Rückfluß erhitzt. Nach dem Abkühlen auf Raumtemperatur wird das Reaktionsgemisch filtriert und unter vermindertem Druck eingeengt. Man nimmt den Rückstand in Toluol auf, schüttelt mit verdünnter, wäßriger Salzsäure aus, trocknet die organische Phase und zieht das Lösungsmittel unter vermindertem Druck ab Der verbleibende Rückstand wird einer fraktionierten Destillation unterworfen. Dabei erhält man 56,9 g eines Öles, das gemäß Gaschromatogramm zu 90 % aus 1-Cyano-cyclopropyl-2-fluor-benzyl-keton besteht. Die Ausbeute errechnet sich danach zu 84 % der Theorie.

Beispiel 2

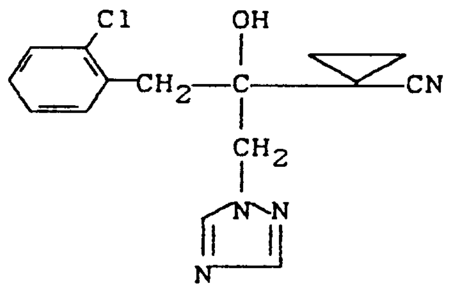

(I-2)

Nach der im Beispiel 1 angegebenen Methode wird auch die Verbindung (I-2) hergestellt.
$^1$H-NMR (200 MHz, $CDCl_3$) : $\delta$ = 0,3-1,1(m;4H), 3,2 (AB-System; 2H), 4,5 (AB-System; 2H), 7,1-7,5 (m; 4H), 7,96 (s; 1H), 8,31 (s; 1H).

Herstellung von Ausgangssubstanzen:

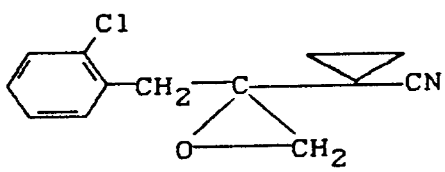

(II-2)

In eine Suspension von 5 g (22,76 mmol) 1-Cyanocyclopropyl-2-chlorbenzyl-keton und 3,22 g (25,04 mmol) Trimethylsulfoxoniumchlorid in 25 ml Toluol werden 10 ml 45 %ige wäßrige Natronlauge bei Raumtemperatur innerhalb 15 Minuten eingetropft. Nach beendeter Zugabe wird das Reaktionsgemisch drei Stunden bei Raumtemperatur und eine Stunde bei 40 °C nachgerührt. Das Reaktionsgemisch wird mit Wasser verdünnt, und die Phasen werden getrennt. Die wäßrige Phase wird dreimal mit Cyclohexan extrahiert. Die vereinigten organischen Extrakte werden einmal mit Wasser gewaschen, über Natriumsulfat getrocknet und unter vermindertem Druck eingeengt. Das Rohprodukt wird ohne zusätzliche Reinigung für die weitere Umsetzung verwendet.

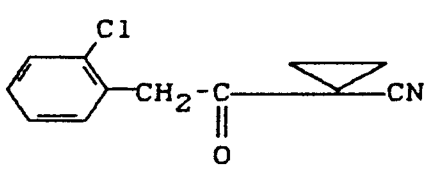

(IV-2)

Ein Gemisch aus 32,7 g (0,5 Mol) Zinkpulver, 56,4 g (0,35 Mol) 2-Chlor-benzyl-chlorid und 375 ml trockenem Ethylenglykol-dimethylether wird unter Stickstoffatmosphäre 2 Stunden unter Rückfluß erhitzt.

Anschließend wird das Reaktionsgemisch unter Stickstoff filtriert. Das Filtrat wird mit 38,9 g (0,3 Mol) 1-Cyano-cyclopropyl-carbonsäurechlorid und 31 mg (0,01 Mol-%) Bis-(triphenylphosphin)-palladium(II)-chlorid versetzt und unter Stickstoffatmosphäre 2 Stunden unter Rückfluß erhitzt. Nach dem Abkühlen auf Raumtemperatur wird das Reaktionsgemisch filtriert und unter vermindertem Druck eingeengt. Man nimmt den Rückstand in Toluol auf, schüttelt mit verdünnter, wäßriger Salzsäure aus, trocknet die organische Phase und zieht das Lösungsmittel unter vermindertem Druck ab. Der verbleibende Rückstand wird einer fraktionierten Destillation unterworfen. Dabei erhält man 58,7 g eines Öles, das gemäß Gaschromatogramm zu 90 % aus 1-Cyano-cyclopropyl-2-chlorbenzyl-keton besteht, Die Ausbeute errechnet sich danach zu 80 % der Theorie. In den folgenden Verwendungsbeispielen wurden die Verbindungen der nachstehend aufgeführten Formeln als Vergleichssubstanzen eingesetzt:

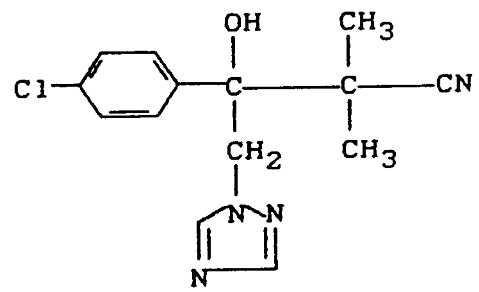
= (A)

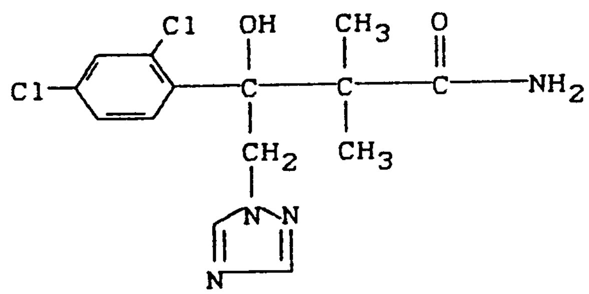
= (B)

(Bekannt aus EP-OS 0 106 515)

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-2) bei einer Konzentration von 250 ppm in der Spritzflüssigkeit einen Wirkungsgrad von 100 %.

Beispiel B

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-2) bei einer Konzentration von 250 ppm in der Spritzflüssigkeit einen Wirkungsgrad von 100 %, während die Vergleichssubstanz (A) einen Wirkungsgrad von 85 % aufweist.

Beispiel C

Fusarium nivale (var. nivale)-Test (Weizen) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf prothektive Wirksamkeit besprüht man junge Pflanzen mit der Wirkstoffzubereitung taufeucht. Nach Antrocknen des Spritzbelages werden die Pflanzen mit einer Konidiensuspension von Fusarium nivale (var. vivale) besprüht.

Die Pflanzen werden in einem Gewächshaus unter lichtdurchlässigen Inkubationshauben bei einer Temperatur von ca. 15 °C und einer relativen Luftfeuchtigkeit von ca. 100 % aufgestellt.

4 Tage nach der Inokulation erfolgt die Auswertung.

In diesem Test zeigt die erfindungsgemäße Verbindung (I-2) bei einer Konzentration von 250 ppm in der Spritzflüssigkeit einen Wirkungsgrad von 100 %, während die Vergleichssubstanz (A) keine Wirkung aufweist.

Beispiel D

Fusarium nivale (var. majus)-Test (Weizen) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser aufdie gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Fusarium nivale (var. Majus) besprüht.

Die Pflanzen werden in einem Gewächshaus unter lichtdurchlässigen Inkubationshauben bei einer Temperatur von ca. 15 °C und einer relativen Luftfeuchtigkeit von ca. 100 % aufgestellt.

4 Tage nach der Inokulation erfolgt die Auswertung.

In diesem Test zeigt die erfindungsgemäße Verbindung (I-2) bei einer Konzentration von 250 ppm in der Spritzflüssigkeit einen Wirkungsgrad von 100 %, während die Vergleichssubstanz (A) keine Wirkung aufweist.

Beispiel E

Fusarium culmorum-Test (Weizen) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Fusarium culmorum besprüht.

Die Pflanzen werden in einem Gewächshaus unter lichtdurchlässigen Inkubationshauben bei einer Temperatur von ca. 20 ° C und einer relativen Luftfeuchtigkeit von ca. 100 % aufgestellt.

4 Tage nach der Inokulation erfolgt die Auswertung.

In diesem Test zeigt die erfindungsgemäße Verbindung (I-2) bei einer Konzentration von 250 ppm in der Spritzflüssigkeit einen Wirkungsgrad von 100 %, während die Vergleichssubstanz (A) keine Wirkung aufweist.

Beispiel F

Uncinula-Test (Rebe) / protektiv

Lösungsmittel: 4,7 Gew.-Teile Aceton
Emulgator: 0,3 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden anschließend bei 23 bis 24 ° C und bei einer relativen Luftfeuchtigkeit von ca. 75 % im Gewächshaus aufgestellt.

14 Tage nach der Inokulation erfolgt die Auswertung.

In diesem Test zeigt die erfindungsgemäße Verbindung (I-1) bei einer Konzentration von 1 ppm in der Spritzflüssigkeit einen Wirkungsgrad von 100 %.

Beispiel G

Pyricularia-Test (Reis) / protektiv

Lösungsmittel: 12,5 Gew.-Teile Aceton
Emulgator: 0,3 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach Antrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 ° C aufgestellt.

4 Tage nach der Inokulation erfolgt die Auswertung.

In diesem Test zeigt die erfindungsgemäße Verbindung (I-1) und (I-2) bei einer Konzentration von 0,025 % in der Spritzflüssigkeit einen Wirkungsgrad von 80 % und mehr, während die Vergleichssubstanz (A) einen Wirkungsgrad von 30 % aufweist.

Beispiel H

Pyricularia-Test (Reis) / systemisch

Lösungsmittel: 12,5 Gew.-Teile Aceton
Emulgator: 0,3 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Tempertur von 25°C und einer rel. Luftfeuchtigkeit von 100 % bis zur Auswertung.

4 Tage nach der Inokulation erfolgt die Auswertung des Krankheitsbefalls.

In diesem Test zeigen die erfindungsgemäßen Verbindungen (I-1) und (I-2) bei einer Aufwandmenge von 100 mg/100 $cm^2$ einen Wirkungsgrad von 80 % und mehr, während die Vergleichssubstanz (A) einen Wirkungsgrad von 70 % aufweist.

Beispiel I

Leptosphaeria nodorum-Test (Weizen) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung taufeucht. Nach Antrocknen des Spritzbelages werden die Pflanzen mit einer wäßrigen Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer rel. Luftfeuchtigkeit von ca. 80 % aufgestellt.

10 Tage nach der Inokulation erfolgt die Auswertung.

In diesem Test zeigt die erfindungsgemäße Verbindung (I-2) bei einer Konzentration von 250 ppm in der Spritzflüssigkeit einen Wirkungsgrad von 85 %, wahrend die Vergleichssubstanz (A) einen Wirkungsgrad von 18 % aufweist.

Beispiel K

Pyrenophora teres-Test (Gerste) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer rel. Luftfeuchtigkeit von ca. 80 % aufgestellt.

7 Tage nach der Inokulation erfolgt die Auswertung.

In diesem Test zeigt die erfindungsgemäße Verbindung (I-2) bei einer Konzentration von 250 ppm in der Spritzflüssigkeit einen Wirkungsgrad von über 70 %, während die Vergleichssubstanz (A) keine Wirkung aufweist.

## Patentansprüche

**1.** Azolylmethyl-cyclopropyl-Derivate dar Formel

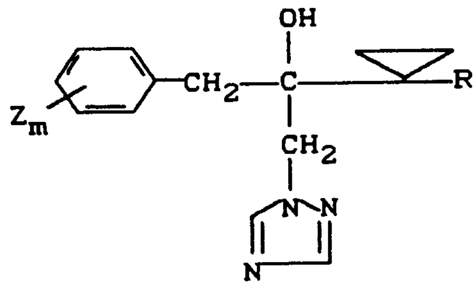

(I)

in welcher

R für Cyano oder eine Gruppe der Formel

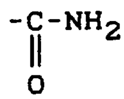

steht,

Z für Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen oder für Alkoximinoalkyl mit 1 bis 4 Kohlenstoffatomen in der Alkoxygruppe und 1 bis 4 Kohlenstoffatomen in der Alkylgruppe steht und

m für die Zahlen 0, 1 oder 2 steht,

sowie deren Säureadditions-Salze und Metallsalz-Komplexe.

**2.** Azolylmethyl-cyclopropyl-Derivata der Formel (I) gemäß Anspruch 1, in denen

R für Cyano oder eine Gruppe der Formel

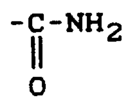

steht,

Z für Fluor, Chlor, Alkyl mit 1 oder 2 Kohlenstoffatomen, Halogenalkyl mit 1 oder 2 Kohlenstoffatoman und 1 bis 3 gleichen oder verschiedenen Halogenatomen, Alkoxy mit 1 oder 2 Kohlenstoffatomen, Halogenalkoxy mit 1 oder 2 Kohlenstoffatoman und 1 bis 3 gleichen oder verschiedenen Halogenatomen oder für Alkoximinoalkyl mit 1 oder 2 Kohlanstoffatomen in der Alkoxygruppe und 1 oder 2 Kohlenstoffatomen in der Alkylgruppe steht und

m für die Zahlen 1 oder 2 steht.

**3.** Verfahren zur Herstellung von Azolylmethyl-cyclopropyl-Derivatendar Formel

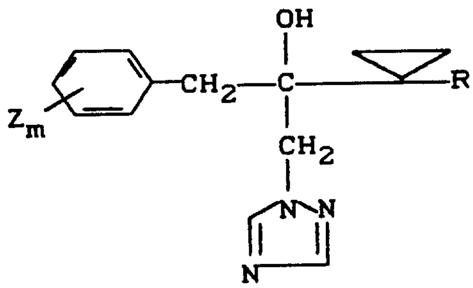

(I)

in welcher
R für Cyano oder eine Gruppe der Formel

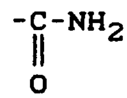

steht,
Z für Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen oder für Alkoximinoalkyl mit 1 bis 4 Kohlenstoffatomen in der Alkoxygruppe und 1 bis 4 Kohlenstoffatomen in der Alkylgruppe steht und
m für die Zahlen 0, 1 oder 2 steht,
sowie von deren Säureadditions-Salzen und Metallsalz-Komplexen, dadurch gekennzeichnet, daß man in einer ersten Stufe Oxirane der Formel

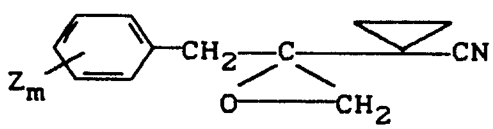

(II)

in welcher
Z und m die oben angegebenen Bedeutungen haben,
mit 1,2,4-Triazol der Formel

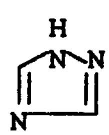

(III)

gegebenenfalls in Gegenwart eines Säurebindemittels und in Gegenwart eines Verdünnungsmittels umsetzt, und gegebenenfalls in einer zweiten Stufe die dabei entstehenden Verbindungen der Formel

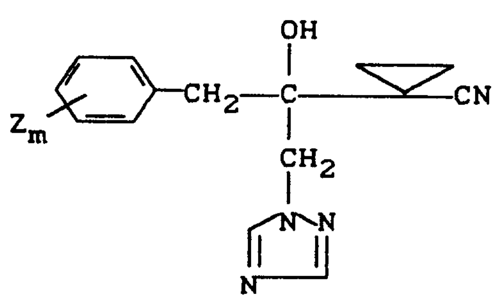

(Ia)

in welcher

Z und m die oben angegebenen Bedeutungen haben,

mit Wasserstoffperoxid in Gegenwart eines inerten organischen Verdünnungsmittels und in Gegenwart einer wäßrigen Alkalilauge sowie in Gegenwart eines Phasentransfer-Katalysators behandelt, und gegebenenfalls anschließend an die so erhaltenen Verbindungen der Formel (I) eine Säure oder ein Metallsalz addiert.

**4.** Fungizide Mittel, gekennzeichnet, durch einen Gehalt an mindestens einem Azolylmethyl-cyclopropyl-Derivat der Formel (I) gemäß Anspruch 1 bzw. an einem Säureadditions-Salz oder Metallsalz-Komplex eines Azolylmethyl-cyclopropyl-Derivates der Formel (I).

**5.** Verwendung von Azolylmethyl-cyclopropyl-Derivaten der Formel (I) gemäß Anspruch 1 bzw. von deren Säureadditions-Salzen und Metallsalz-Komplexen zur Bekämpfung von Pilzen.

**5.** Verfahren zur Bekämpfung von Pilzen, dadurch gekennzeichnet, daß man Azolylmethyl-cyclopropyl-Derivate der Formel (I) gemäß Anspruch 1 bzw. deren Säureadditions-Salze oder Metallsalz-Komplexe auf die Pilze und/oder deren Lebensraum ausbringt.

**7.** Verfahren zur Herstellung von fungiziden Mitteln, dadurch gekennzeichnet, daß man Azolylmethyl-cyclopropyl-Derivate der Formel (I) gemäß Anspruch 1 bzw. deren Metallsalz-Komplexe oder Säureadditions-Salze mit Streckmitteln und/oder oberflächenaktiven Stoffen vermischt.

**8.** Oxirane der Formel

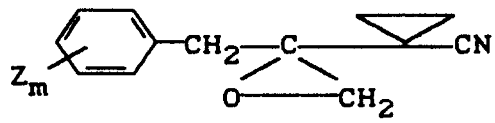

(II)

in welcher

Z für Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen oder für Alkoximinoalkyl mit 1 bis 4 Kohlenstoffatomen in der Alkoxygruppe und 1 bis 4 Kohlenstoffatomen in der Alkylgruppe steht und

m für die Zahlen 0, 1 oder 2 steht.

**9.** Verfahren zur Herstellung von Oxiranen der Formel

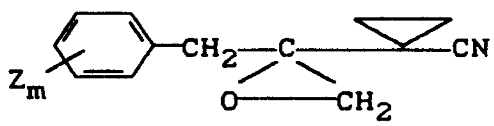

(II)

in welcher

Z für Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen oder für Alkoximinoalkyl mit 1 bis 4 Kohlenstoffatomen in der Alkoxygruppe und 1 bis 4 Kohlenstoffatomen in der Alkylgruppe steht und

m für die Zahlen 0, 1 oder 2 steht,

dadurch gekennzeichnet, daß man Benzylketone der Formel

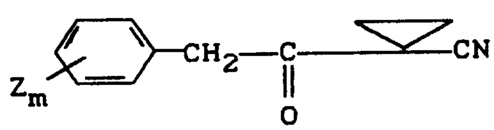

(IV)

in welcher

Z und m die oben angegebenen Bedeutungen haben,

entweder

α) mit Dimethyloxosulfonium-methylid der Formel

$$\overset{\delta^{\oplus}\,\delta^{\ominus}}{(CH_3)_2SOCH_2} \qquad (V)$$

oder

$\beta$) mit Dimethylsulfonium-methylid der Formel

$$\overset{\delta^{\oplus}\,\delta^{\ominus}}{(CH_3)_2S\ CH_2} \qquad (VI)$$

in Gegenwart eines Verdünnungsmittels umsetzt.